(19) Europäisches Patentamt
European Patent Office
Office européen des brevets

(11) Publication number: **0 040 254**
**B1**

(12) **EUROPEAN PATENT SPECIFICATION**

(45) Date of publication of patent specification: **13.07.83**

(51) Int. Cl.³: **C 07 C 101/26,**
C 07 C 51/02

(21) Application number: **80102737.6**

(22) Date of filing: **16.05.80**

(54) **Process for preparing carboxylic acids by reaction of alkali metal carboxylates and liquid cation exchange agents.**

(43) Date of publication of application:
**25.11.81 Bulletin 81/47**

(45) Publication of the grant of the patent:
**13.07.83 Bulletin 83/28**

(84) Designated Contracting States:
**DE FR GB NL SE**

(56) References cited:
**US - A - 2 895 989**

**CHEMICAL ABSTRACTS, vol. 71, no. 5, 4th August 1969, page 266, no. 25157k, Columbus, Ohio, U.S.A., I. KOJIMA et al.: "Extraction of copper (II) and sodium with bis (2-ethylhexyl) hydrogen phosphate"**

**C.R. ACAD. SC. PARIS, Série C, vol. 267, 18th November 1968, pages 1373—1376, Montreuil, FR., Y. MOGNO et al.: "Etude du schéma d'extraction des métaux alcalins et alcalino-terreux par les acides di-alcoylphosphoriques"**

(73) Proprietor: **THE DOW CHEMICAL COMPANY**
**Dow Center 2030 Abbott Road Post Office Box 1967**
**Midland Michigan 48640 (US)**

(72) Inventor: **Wilson, David Alan**
**229 San Saba**
**Richwood, Texas (US)**
Inventor: **Schmidt, Roy Wilbur**
**941 Magnolia**
**Lake Jackson, Texas (US)**

(74) Representative: **Weickmann, Heinrich, Dipl.-Ing. et al,**
**Postfach 860 820 Möhlstrasse 22**
**D-8000 München 86 (DE)**

(56) References cited:
**CHEMICAL ABSTRACTS, vol. 78, no. 10, 12th March 1973, page 305, no. 62933a, Columbus, Ohio, U.S.A., V.P. LANIN et al.: "Extraction of sodium, rubidium, and cesium from perchlorate solutions by di-2-ethylhexylphosphoric acid"**

**EP 0 040 254 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

# Process for preparing carboxylic acids by reaction of alkali metal carboxylates and liquid cation exchange agents

This invention relates to the preparation of a carboxylic acid from the corresponding alkali metal or ammonium carboxylate in an aqueous solution. In particular, this invention relates to the use of liquid ion-exchange agents for the conversion of salts of carboxylic acids into the corresponding carboxylic acids.

Among the various methods previously used for the recovery of carboxylic acids from their salts, the most common has been acidification with an aqueous solution of a mineral acid. This method has not been entirely satisfactory for the recovery of water-soluble carboxylic acids or aminopolycarboxylic acids, which are chelating agents. The water-soluble carboxylic acids are difficult to separate from the salts of mineral acids.

US—A—2 895 989 describes a method for transforming the alkaline earth and alkali metal salts (including ammonium salts) of iminodiacetic acid into free iminodiacetic acid by contacting a solution of the salts with a fixed bed of a sulfonated vinyl aromatic ion exchange resin.

The practice of this invention is useful to produce in high yield carboxylic acids free of contaminant by-product salts produced in the conventional acidification reactions.

The present invention is a process for preparing a carboxylic acid from its corresponding alkali metal carboxylate, comprising:

(a) reacting an aqueous solution of the alkali metal or ammonium carboxylate with a liquid cation-exchange agent in a water-insoluble organic solvent which does not dissolve the corresponding carboxylic acid;

(b) separating the aqueous solution containing the thus-prepared carboxylic acid from the organic solvent.

The practice of the present invention shows that despite the high alkalinity of solutions of ammonium or alkali metal carboxylates, liquid cation-exchange agents effectively acidify these alkali metal carboxylates.

The carboxylates acidified in this process can be essentially any carboxylate which is soluble in water and which is substantially insoluble in the organic solvent for the liquid ion-exchange agent. This process is applicable to alkali metal or ammonium salts of aliphatic hydroxycarboxylic acids or condensates thereof, for example, an alkali metal glycolate or an alkali metal salt of diglycolic acid. The practice of this process is particularly applicable to an alkali metal or ammonium salt of an iminopolycarboxylic acid or of an aminopolycarboxylic acid, such as alkali metal salts of nitrilotriacetic acid (NTA), ethylenediaminetetraacetic acid (EDTA), diethylenetriaminepentaacetic acid (DTPA), triethylenetetraaminehexaacetic acid (TTHA), hydroxyethyliminodiacetic acid (HEIDA), N,N-di(2-hydroxyethyl)glycine (DHEG), glycine, iminodiacetic acid (IDA) and N-(2-hydroxyethyl)ethylenediaminetriacetic acid (HEDTA). The practice of this invention is most applicable to those alkali metal salts of aminopolycarboxylic acid, such as DTPA and HEDTA, which are likely to super-saturate rather than precipitate from a concentrated aqueous solution.

The term alkali metal carboxylate as used herein refers, generically, to ammonium carboxylate as well as lithium, potassium and sodium carboxylates. The preferred alkali metal carboxylate is a sodium carboxylate.

It is operable to employ a mixture of alkali metal carboxylate compounds and in particular a variety of alkali metal ions in a single acidification. It is also operable in this process to employ a compound which bears carboxylic acid moieties as well as alkali metal carboxylate moieties. To illustrate, it is advantageous that the trisodium salt of HEDTA is the major component when acidification is initiated, but it is operable to initiate acidification when the trisodium, disodium or monosodium salt of HEDTA is the major component present. Of course, as the acidification progresses the major component is progressively one more highly acidified.

The carboxylic acid prepared by this method can bear alkali metal carboxylate moieties as well as carboxylic acid moieties. The only requirement is that the carboxylic acid is acidified to a greater degree than the alkali metal carboxylate from which it is prepared. However, it is desirable that the carboxylic acid is substantially completely acidified by this process.

The alkali metal carboxylates can be prepared by a variety of conventional means, for example, reaction of esters with alkali metal hydroxides. U.S. Patent Nos. 2,387,735, Bersworth, issued October 30, 1945, and 2,407,645, Bersworth, issued September 17, 1946, are of interest in that they describe methods of preparing sodium aminopolycarboxylates. Many alkali metal carboxylate compounds of interest are also available commercially.

The alkali metal carboxylate is present in an aqueous solution and its concentration is not critical. Advantageously, the alkali metal carboxylate is present in great enough concentration in the aqueous solution, so that the acidification can be conducted efficiently. Desirably, the aqueous product solution is of such concentration that it can be effectively and economically used as a chelating solution. Of course, the preferred range of alkali metal carboxylate concentration depends on the specific compound, but normally concentrations of from 10 to 50 weight percent are preferred.

The organic solvent should be liquid under conditions of use and inert to the liquid ion-exchange

agent, the alkali metal carboxylate and its corresponding acid. The organic solvent should also be insoluble in water (as classified in *The Handbook of Chemistry and Physics* or within the definition of insoluble as used by said handbook), so that a clean separation of the organic and the aqueous phases can be made after contact. Finally, the carboxylic acid product must be insoluble or substantially insoluble in the organic solvent to protect the yield of the acid product. Suitable organic solvents generally include kerosene, benzene, carbon tetrachloride and perchloroethylene, with kerosene being the preferred solvent. Certain solvents, such as methylene chloride, are operable, but not desirable because of the poor separation effected between the organic and aqueous phases.

The liquid cation-exchange agent can be any such agent which is insoluble or only slightly soluble in water and does not form emulsions with water and which reacts selectively with an alkali metal carboxylate to produce the corresponding carboxylic acid in the aqueous solution in high yield. Preferably, the liquid cation-exchange agent should be a phosphoric acid derivative corresponding to the formula

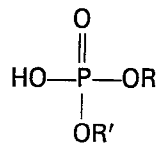

wherein R and R′ are each hydrogen, alkyl, aralkyl, alkaryl or aryl, providing that the total number of carbon atoms in R plus R′ is at least one. In general, it is desirable that R and R′ are alkyl groups independently having from 1 to 20 carbon atoms. Generally, mixtures of operable liquid cation-exchange agents can also be employed. Di(2-ethylhexyl)phosphoric acid (D2EHPA) is the preferred liquid cation-exchange agent.

The concentration of the liquid cation-exchange agent is not critical. However, the agent generally produces a higher degree of acidification for a given amount of the agent as the concentration of the agent in the organic solvent is increased. The aforementioned effect diminishes at higher concentrations of the agent. Of course, the preferred range of liquid cation-exchange agent concentration depends on the specific agent, the organic solvent and the alkali metal carboxylate as well as other factors, but typically a concentration of the liquid cation-exchange agent of from 10 to 70 percent by weight of the organic solution is preferred.

The aqueous solution of alkali metal carboxylate is contacted with the liquid ion-exchange agent in the organic solution in any manner which produces a suitable transfer of ions from the aqueous to the organic phase. To illustrate, Kunin in *Angew. Chem-Int'l Ed.,* 1:149—155 (1963) discloses a variety of suitable liquid-liquid contactors. The contact can occur as a batch or a continuous process. Repeated contact of the alkali metal carboxylate with fresh portions of the liquid ion-exchange agent can be used in a batchwise process to produce higher degrees of acidification. Advantageously, the contact can be effected by continuous countercurrent contact of the organic and aqueous phases in a suitable column contactor to promote substantially complete acidification with a minimal excess of liquid ion-exchange agent.

The equivalent weight ratio of the liquid cation-exchange agent to the alkali metal carboxylate is also important to the degree of acidification effected. When contact of the two reactants occurs in a batch operation, as opposed to a continuous operation, a relatively greater excess of the liquid cation-exchange agent must be employed to achieve the same degree of acidification. An equivalent weight ratio (exchange agent:carboxylate) of less than 1:1 is operable, but not desirable because complete acidification is not effected. An equivalent weight ratio greater than 50:1 is operable, but not desirable even in a batch operation because of the diminishing increase in effectiveness at higher ratios and the uneconomic use of the exchange agent at these ratios.

The temperature during contact is not critical so long as the carboxylate and ion-exchange reactants remain in their respective solutions and do not undergo significant decomposition. It is normally advantageous to contact the aqueous and organic solutions at a temperature of from 20°C to 80°C, preferably from 20°C to 50°C. However, a higher temperature may be desirable to prevent the acid product from prematurely precipitating.

Pressure during contact of from subatmospheric pressures to 1000 pounds per square inch gauge ($70.5 \times 10^5$Pa) is operable, with atmospheric pressure generally being preferred for reasons of convenience.

The degree of acidification can conveniently be determined from the measurement of the pH of the aqueous solution. The relative distribution of ionic species of the carboxylic acid in the solution can then be determined from the pertinent acid dissociation constants.

After the desired degree of acidification is effected, the aqueous phase containing the carboxylic acid can be separated from the organic phase by any convenient means known to the art. The separated organic phase can be extracted with fresh water to recover residual amounts of the carboxylic acid, but this is generally not necessary because of the low solubility of the acid in a properly selected organic solvent. Likewise, extraction of the separated aqueous phase with fresh organic solvent can be employed to remove residual impurities, but is generally not necessary.

The carboxylic acid product can be conveniently isolated by precipitating it from a hot, concentrated aqueous solution by cooling the solution. This method isolates the carboxylic acid substantially free of alkali metal carboxylates. Alternatively, the water can be evaporated or removed by distillation to isolate the carboxylic product as a residue. While the foregoing methods of recovering the acid product are convenient, the instant acidification process does not extend to and therefore is not limited by any particular method of product isolation.

When the liquid ion-exchange agent is used in a large molar excess relative to the carboxylate, it can be used repeatedly prior to regeneration, because only a minor part of the agent present is exhausted in each acidification. The exchange agent can be regenerated in a batch or a continuous process by contacting the agent in the separated organic solution with a concentrated aqueous solution of a strong acid, such as hydrochloric acid or sulfuric acid. Conveniently, in a batch process, an aqueous solution of a strong mineral acid is brought together and agitated with an equimolar or lesser amount of partially exhausted ion-exchange agent in its organic solution, so as to regenerate the exchange agent. Alternatively, the partially exhausted liquid ion-exchange agent in the organic solution can, in a continuous process, be contacted countercurrently with an aqueous solution of a strong mineral acid to effect regeneration of the liquid ion-exchange agent. Desirably, the aqueous solution of mineral acid is employed in a concentration of at least 1 mole per liter. The partially exhausted exchange agent can be regenerated to greater than 95 percent of the acid form present in the fresh exchange agent by the aforementioned method. The regenerated exchange agent can be employed in the instant method of acidification.

The following examples illustrate the invention.

Example 1

In a series of runs which differed only in the organic solvent, an aqueous solution containing 20 percent by weight of trisodium N-(2-hydroxyethyl)-ethylenediaminetriacetate ($Na_3HEDTA$) having a pH of 13.2 is mixed with an organic solution containing 40 percent by weight of D2EHPA to create a reaction mixture. This reaction mixture contains an equivalent weight ratio of D2EHPA to $Na_3HEDTA$ of 10:1. The aqueous solution and organic solution are contacted with agitation at a temperature of 22°C.

After one hour, the agitation is stopped and the organic and aqueous solutions are allowed to separate into phases. If the organic and aqueous phases are clearly defined and readily separable, the aqueous solution is separated from the organic solution. The pH of the aqueous solution is then measured.

The pH of the separated aqueous solution is adjusted to 11.0 and the chelation value determined by titration with a standardized solution of calcium chloride in the presence of oxalate ions. The recovery of chelation value is calculated by comparing the total amount of chelant (as determined by the calcium titration) before reaction with the liquid ion-exchange agent with the amount of chelant present in the separated aqueous phase after the reaction. The final pH and percent chelation recovery for contact with each of the organic solvents evaluated is tabulated in Table I.

TABLE I

| Solvent | % Chelation Recovery | Final pH |
|---|---|---|
| Kerosene | 99.6 | 3.55 |
| Carbon Tetrachloride | 98.2 | 3.63 |
| Perchloroethylene | 99.2 | 3.56 |
| Dichloromethane | * | 3.75 |

* Poor separation of phases, no titration made.

The pH data in Table I indicate that substantial acidification of the $Na_3HEDTA$ occurs in all of the solvents. However, the pH of a saturated aqueous solution of HEDTA at 25°C is 2.2. Based on the acid dissociation constant of HEDTA, this batch process in kerosene, carbon tetrachloride and perchloroethylene completely acidifies about 20 percent of the $Na_3HEDTA$ and produces essentially a remaining amount of the monosodium salt of HEDTA.

The recovery of chelation value in acidifications conducted with three of the solvents indicate that little of the $Na_3HEDTA$ or the acidified products is lost to the organic phase. The methylene chloride solvent is less desirable than the other three solvents, because a clean separation between the phases is not obtained.

Example 2

In a manner otherwise similar to the method set out in Example 1, a 20 weight percent solution of $Na_3HEDTA$ is acidified with a variety of liquid cation-exchange agents. In the tabulated separate runs, the aqueous solution of $Na_3HEDTA$ is contacted with kerosene solutions of D2EHPA, oleic acid, or a 1:1 mixture by weight of D2EHPA and mono-2-ethylhexyl phosphoric acid (M2EHPA). In two more runs, dinonylnaphthalene sulfonic acid (DNNSA) in heptane is employed as the liquid cation-exchange agent in the organic solvent. The final pH, the equivalent weight ratio of the exchange agent to $Na_3HEDTA$, weight percent of the agent in the organic solvent, and percent chelation recovery for contact with each of the exchange agents are tabulated in Table II.

TABLE II

| Exchange Agent | Eq. Ratio (Exch. Ag.:—$Na_3HEDTA$) | % Conc Exch. Agent | % Chelation Recovery | Final pH |
|---|---|---|---|---|
| D2EHPA | 2:1 | 40 | 97.9 | 4.6 |
| D2EHPA | 10:1 | 40 | 99.6 | 3.6 |
| Oleic Acid | 5:1 | 13 | —[1] | 7.2 |
| Oleic Acid | 10:1 | 13 | 90.0 | 7.4 |
| D2EHPA/M2EHPA | 1:1 | 43 | 80.2[2] | 4.7 |
| DNNSA | 1:1 | 20 | —[3] | —[3] |
| DNNSA | 10:1 | 20 | —[3] | —[3] |

[1] Very poor separation of layers; emulsion formed.
[2] Very slow phase separation.
[3] Loss of chelant into organic phase; emulsion formed.

The pH data in Table II indicate that the phosphoric acid derivatives are relatively more efficient exchange agents in this embodiment than are the other agents tested.

The recovery of chelation values with the four exchange agents tabulated indicates that D2EHPA is superior in this respect to the other liquid ion-exchange agents.

Example 3

An aqueous solution containing 20 percent by weight of $Na_3HEDTA$ is reacted in separate acidifications with solutions of 10, 40 and 70 percent by weight D2EHPA in kerosene. The equivalent weight ratio of D2EHPA to $Na_3HEDTA$ is varied for each concentration of D2EHPA in the ratios of 1:1, 2:1, 10:1 and 30:1. The manner of acidification is otherwise similar to that set out in Example 1. After the acidification the pH of the aqueous solution is measured. The equivalent weight ratio, weight percent of D2EHPA in kerosene and the final pH are tabulated in Table III.

TABLE III

| Equivalent Ratio ($D2EHPA:Na_3HEDTA$) | Final pH at Listed Concentration of D2EHPA | | |
|---|---|---|---|
| | 10% | 40% | 70% |
| 1:1 | 6.20 | 5.50 | 5.45 |
| 2:1 | 5.20 | 4.65 | 4.70 |
| 10:1 | 4.05 | 3.60 | 3.45 |
| 30:1 | 3.65 | 3.10 | 3.05 |

The pH data in Table III indicate that for a given equivalent weight ratio of D2EHPA to $Na_3HEDTA$, a greater degree of acidification is effected with 40 than with 10 weight percent D2EHPA. However, there is only a slight increase in the degree of acidification when the concentration of D2EHPA is 70 instead of 40 weight percent. Further, the data in Table I indicate a significant increase in the degree of acidification at all concentrations of D2EHPA as the equivalent ratio of D2EHPA to $Na_3HEDTA$ increases.

Example 4

An aqueous solution containing 20 or 40 percent by weight of $Na_3HEDTA$ is reacted in separate acidifications with solutions of 40 and 70 percent by weight D2EHPA in kerosene. The equivalent weight ratio of D2EHPA to $Na_3HEDTA$ is 10:1 in each instance. The manner of acidification is otherwise similar to that set out in Example 1. After the acidification the pH of the aqueous solution is measured. The aqueous phase is then separated from the organic phase and the chelation value determined in the conventional manner. The initial weight percent of the $Na_3HEDTA$ and of D2EHPA, the final pH of the aqueous solution and the recovery of chelation value are tabulated in Table IV.

TABLE IV

| $Na_3HEDTA$ (%) | D2EHPA (%) | Final pH | Recovery of Chelation Value (%) |
|---|---|---|---|
| 20 | 40 | 3.60 | 99.6 |
| 40 | 40 | * | 72.9 |
| 20 | 70 | 3.45 | 97.0 |
| 40 | 70 | * | 92.4 |

* Product crystallized before pH measurement obtained.

The data in Table IV indicate that at a concentration of 40 percent by weight $Na_3HEDTA$, the acid product formed precipitates very rapidly. This rapid precipitation results in the loss of some of the acid product on the separatory funnel, thereby resulting in a lower recovered chelation value. Separation at higher temperatures eliminates this problem.

Example 5

A reciprocating plate column 6 feet (1.83 m) in length and having a 1 inch (2.54 cm) internal diameter is used to effect continuous countercurrent contact of an aqueous solution of $Na_3HEDTA$ and a kerosene solution of D2EHPA. An aqueous solution containing 41.3 percent by weight $Na_3HEDTA$ is introduced into the top of the column. Into the bottom of the column is introduced a solution of kerosene containing 60 percent by weight D2EHPA. The rate of introduction of the two reactants is adjusted in separate runs to effect various equivalent weight ratios and various amounts of reactants put through the column in units of gallons per hour per square foot ($gph/ft^2$) ($1/hr/m^2$). The strokes per minute of the reciprocating plate are optimized in each run to obtain the lowest pH measurement for the aqueous product stream. A final pH measurement of the aqueous product stream is taken at the end of the run. The column and solutions of reactants are heated in order to maintain the solubility of the acid within the reaction zone of the column. The relevant parameters and operating conditions are tabulated in Table V.

TABLE V

| Solution Throughput, gph/ft² (1/hr/m²) | Equivalent Ratio (D2EHPA/-$Na_3$HEDTA) | Temperature (°C) | Run Time (hours) | Final pH |
|---|---|---|---|---|
| 600 (24,600) | 3.3:1 | 48 | 2.0 | 2.50 |
| 600 (24,600) | 3.3:1 | 58 | 2.0 | 2.54 |
| 600 (24,600) | 3.3:1 | 68 | 2.0 | 2.59 |
| 600 (24,600) | 3.3:1 | 78 | 3.0 | 2.59 |
| 600 (24,600) | 1.3:1 | 60 | 2.0 | 2.90 |
| 600 (24,600) | 2.3:1 | 60 | 3.0 | 2.50 |
| 600 (24,600) | 4.3:1 | 60 | 3.0 | 2.49 |
| 900 (36,900) | 1.3:1 | 60 | 2.3 | 3.18 |
| 900 (36,900) | 2.3:1 | 60 | 3.0 | 2.88 |
| 900 (36,900) | 3.3:1 | 60 | 2.5 | 2.58 |
| 1200 (49,200) | 3.3:1 | 60 | 2.8 | 2.83 |

At the lowest rate of solution throughout tested (600 gph/ft²), more than 60 percent of the $Na_3$HEDTA is converted to HEDTA in all but one of the runs. The lower conversion in one of the runs is the result of the relatively slight excess of the liquid cation-exchange agent employed in that instance. At higher rates of solution throughout an even greater excess of D2EHPA is necessary to effect the same degree of acidification.

Temperatures of from 48°C to 78°C have little effect on the degree of acidification effected.

Comparative Example 1

In a process not embodying the claimed process, an aqueous solution of $Na_3$HEDTA is acidified with sulfuric acid A 600-gram aqueous solution of 20.7 or 41.3 weight percent $Na_3$HEDTA is cooled to 0°C. Sulfuric acid is then added to the aqueous solution to reduce the pH of the solution to 2.0. The solution is then seeded with a small amount of sodium sulfate crystals to precipitate solids. The solution is filtered by suction. The remaining solids are washed with distilled water at 0°C and this second filtrate is collected. Each filtrate is analyzed to measure the weight percent of sulfate and the percent recovery of chelation value by conventional methods. The data and operating conditions are tabulated in Table VI.

TABLE VI

| | | Filtrate 1 | | Filtrate 2 | |
|---|---|---|---|---|---|
| $Na_3$HEDTA (Weight %) | $H_2O$ Wash (milliliters) | Chelation (% Recovery) | Sulfate (Weight %) | Chelation (% Recovery) | Sulfate (Weight %) |
| 41.3 | 500 | 29.0 | 19.4 | 20.0 | 10.7 |
| 20.7 | 175 | 73.5 | 21.5 | 23.5 | 13.4 |

As is seen from the data in Table VI, the solubility of sodium sulfate is high enough that a large amount remains in the solutions of HEDTA. Therefore, it is difficult to separate the water-soluble carboxylic acid product from the salt of the mineral acid.

## Claims

1. A process for preparing a carboxylic acid from its corresponding alkali metal carboxylate, characterized by:

(a) reacting an aqueous solution of the alkali metal or ammonium carboxylate with a liquid cation-exchange agent, which is insoluble or only slightly soluble in water and does not form emulsions with water and which reacts selectively with an alkali metal carboxylate, in a water-insoluble organic solvent which does not dissolve the corresponding carboxylic acid;

(b) separating the aqueous solution containing the thus-prepared carboxylic acid from the organic solvent.

2. The process as described in Claim 1 and further characterized in that the liquid cation-exchange agent is a phosphoric acid derivative corresponding to the formula

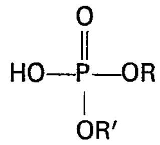

wherein R and R′ are each hydrogen, alkyl, aralkyl, alkaryl or aryl, providing that the total number of carbon atoms in R plus R′ is at least one.

## Patentansprüche

1. Verfahren zur Herstellung einer Carboxylsäure aus dem entsprechenden Alkalimetallcarboxylat, dadurch gekennzeichnet, daß

(a) eine wäßrige Lösung des Alkalimetall- oder -ammoniumcarboxylats mit einem flüssigen Kationenaustauscher, der unlöslich ist oder nur schwer löslich in Wasser und keine Emulsionen mit Wasser bildet und der selektiv mit Alkalimetallcarboxylat reagiert, in einem wasserunlöslichen organischen Lösungsmittel, das die entsprechende Carboxylsäure nicht löst, reagiert;

(b) die wäßrige Lösung, die die so hergestellte Carboxylsäure enthält, von dem organischen Lösungsmittel getrennt wird.

2. Verfahren nach Anspruch 1, dadurch gekennzeichnet, daß der flüssige Kationenaustauscher ein Phosphorsäurederivat ist mit der Formel

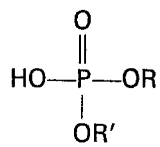

wobei R und R′ jeweils Hydrogen, Alkyl, Aralkyl, Alkaryl oder Aryl bedeuten, vorausgesetzt, daß die Gesamtanzahl an Kohlenstoffatomen in R und R′ mindestens 1 ist.

## Revendications

1. Procédé pour la préparation d'un acide carboxylique à partir de son carboxylate de métal alcalin correspondant, caractérisé en ce qu'il consiste:

(a) à faire réagir une solution aqueuse du carboxylate de métal alcalin ou d'ammonium sur un échangeur liquide de cations, qui est insoluble ou seulement légèrement soluble dans l'eau et ne forme pas d'émulsions avec l'eau, et qui réagit sélectivement avec un carboxylate de métal alcalin, dans un solvant organique insoluble dans l'eau qui ne dissolve pas l'acide carboxylique correspondant;

(b) à séparer du solvant organique la solution aqueuse contenant l'acide carboxylique ainsi préparé.

2. Procédé selon la revendication 1, caractérisé en ce que l'échangeur liquide de cations est un dérivé de l'acide phosphorique correspondant à la formule:

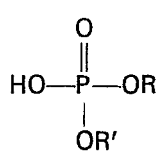

où R et R′ sont chacun l'hydrogène, un alkyle, un aralkyle, un alcaryle ou un aryle, à la condition que le nombre total d'atomes de carbone dans R plus R′ soit d'au moins un.